Europäisches Patentamt

European Patent Office

Office européen des brevets

(19)

(11)  **EP 1 267 295 A1**

(12)  **EUROPEAN PATENT APPLICATION**

(43)  Date of publication:
      **18.12.2002   Bulletin 2002/51**

(51) Int Cl.⁷: **G06F 19/00**

(21)  Application number: **02011654.7**

(22)  Date of filing: **31.05.2002**

(84)  Designated Contracting States:
      **AT BE CH CY DE DK ES FI FR GB GR IE IT LI LU
      MC NL PT SE TR**
      Designated Extension States:
      **AL LT LV MK RO SI**

(30)  Priority: **11.06.2001   US 297299 P**

(71)  Applicant: **WARNER-LAMBERT COMPANY
      Morris Plains New Jersey 07950 (US)**

(72)  Inventors:
      • **Tidwell, Carl
        Chelsea, Michigan 48118 (US)**
      • **Wang, Yixin
        San Diego, Claifornia 92130 (US)**

      • **Wu, Chien-Fu Jeff
        Ann Arbor, Michigan 48105 (US)**
      • **Wu, Peiru
        Ann Arbor, Michigan 48105 (US)**
      • **Xu, Hongquan
        Los Angeles, California 90034 (US)**

(74)  Representative: **Tesch, Rudolf, Dr. et al
      Warner-Lambert Company,
      Legal Division,
      Patent Department,
      c/o Gödecke AG,
      Mooswaldallee 1
      79090 Freiburg (DE)**

(54)  **A method and system for analyzing gene expression data using a smooth response surface algorithm**

(57)     A Smooth Response Surface (SRS) algorithm is provided as a more elaborate data mining technique for analyzing gene expression data, as well as computationally constructing a gene regulatory network. A three-dimensional smooth response surface is generated to capture the biological relationship between the target and activator-repressor. This novel technique is applied to functionally describe triplets of activators, repressors and targets, and their regulations in gene expression data. A diagnostic strategy is built into the SRS algorithm to evaluate the scores of the triplets so that those with low scores are kept and a regulatory network is constructed based on this information and existing biological knowledge. The predictions based on the identified triplets in two yeast expression data agree with experimental data in the literature. The invention provides a novel model with attractive mathematical and statistical features that make the algorithm valuable for mining expression or concentration information in gene expression analysis for determining the function of uncharacterized proteins, as well as for developing a more accurate understanding of coherent regulatory pathways.

**FIG. 1**

**Description**

FIELD OF THE INVENTION

The present invention relates generally to the field of bioinformatics, and more specifically to a system and method for analyzing gene expression data.

BACKGROUND OF THE INVENTION

Cells regulate the expression of their genes in response to environmental changes. Normally this regulation is beneficial to the cell, protecting it from starvation or injury; however, errors in this regulation can lead to serious diseases ranging from cancer to heart disease. Measuring the differential expression of genes from organisms subjected to different stresses, from organisms at varying developmental stages, or from different tissues, provides information instrumental in understanding the relationships between genes and their functions. Gene regulation is useful for both assaying drugs and as a source of new molecular targets, assuming the regulatory network controlling a given gene is well-understood. As such, changes in gene expression patterns can be used to assay drug efficacy throughout the drug discovery process.

One assay that takes advantage of existing sequence information, and that is complementary to sequence and genetic analysis, is gene expression profiling. Expression profiling can be carried out by one of a number of different technologies, such as microarray or GeneChip® technologies, which typically measure the expression level of thousands of genes simultaneously using an array of cDNA clones, PCR products, or oligonucleotides bound to a solid support, such as a glass microscope slide or silicon surface. These arrays are hybridized under stringent conditions with a complex sample representing mRNAs expressed in the test cell or tissue. Target sequences hybridize to immobilized oligonucleotides and are typically detected via fluorescent labels. Relative intensity levels of fluorescent labels indicate relative gene expression in a given sample obtained from a source subjected to a particular condition.

As a sample source is subjected to a variety of conditions, a given gene will display an expression profile under the conditions. Thus, gene expression profiles represent a vector of gene expression intensities corresponding to multi-conditional gene expression patterns. The results from these gene expression profiling technologies are quantitative and highly parallel, thereby allowing an accurate snapshot to be made of the workings of the cell in a particular state. Since thousands of hybridization reactions may occur in a single array, gene expression profiling assays generate huge data sets that are not amenable to simple analysis. To maximize the use of such data, efforts are underway to develop algorithms for interpreting and interconnecting results from different genes under different conditions.

Existing methods for analyzing gene expression data include classical and modern statistical clustering techniques, which group genes with similar expression patterns. Such techniques include methods and applications of hierarchical clustering (Eisen et al., *PNAS,* 1998;96:14863-14868; Spellman et al., *Mol. Biol. Cell*, 1998;9:3273-3297; Alon et al., *PNAS,* 1999;96:6745-6750; Iyer et al., *Science,* 1999;283:83-87; and Perou et al., *PNAS*, 1999;96: 9212-9217), and self-organizing mapping (Toronen et al., *FEBS Letters*, 1999;451:142-146 and Tamayo et al., *PNAS*, 1999;96:2907-2912). However, clustering methods only distinguish between those genes that have the same and different expression profiles. Genes in any given cell make up a complex network that cannot be revealed with such current techniques. In order to determine networks describing how various genes interrelate, more elaborate data mining techniques are needed.

Recently, Woolf and Wang (*Physiol. Genomics.*, 2000;3:9-15) proposed a fuzzy logic approach in order to generate a connected network of genes using only gene expression data. The fuzzy logic algorithm provides a method for transforming precise numbers into qualitative descriptions, which are then analyzed using heuristic rules to generate a qualitative descriptor in the heuristic solution, and finally transformed back into a precise number.

SUMMARY OF THE INVENTION

The present invention generally provides a method and system for managing and analyzing gene expression data using a Smooth Response Surface (SRS) algorithm.

In a first aspect of the invention, a method for managing and analyzing information obtained from differential expression of genetic information in biological cells is provided. In one embodiment, the method can comprise:

receiving input data obtained from gene expression profiling, wherein the input data represents a direction and a magnitude of expression of profiled genes or gene products;

transforming the input data to map the input data onto a three-dimensional response surface and generate putative gene or gene product triplets of interrelated genes or gene products, wherein the three-dimensional response surface describes a regulatory interrelationship among the profiled genes or gene products;

evaluating the transformed data for each putative gene or gene product triplet to determine a lack-of-fit value therefore;

evaluating the lack-of-fit value for selected putative gene or gene product triplets to determine a diagnostic value therefore; and

evaluating the lack-of-fit value and the diagnostic value for at least some of the selected putative gene or gene product triplets to determine an interrelationship score therefore, wherein the interrelationship score reflects the strength of the gene or gene product triplet interrelationship.

In another aspect of the invention, the method can optionally include input data imputation and filtering, as well as gene or gene product regulatory network predictions. The predicted gene or gene product regulatory network may further be at least partially verified using known biological function and/or relationship information.

In yet another aspect of the invention, a system for managing and analyzing information obtained from differential expression of genetic information in biological cells is provided. In one embodiment, the system can comprise:

a first interface to an expression profiling data source for providing input data obtained from gene expression profiling to a computational platform, wherein the input data represents a direction and a magnitude of expression of profiled genes or gene products; and

a computational platform comprising:

an SRS transformation module for mapping expression profiling data onto a three-dimensional response surface to generate putative gene or gene product triplets of interrelated genes or gene products, wherein the three-dimensional response surface describes a regulatory interrelationship among the profiled genes or gene products;

a lack-of-fit determination module for determining a lack-of-fit value for each putative gene or gene product triplet;

a diagnostic strategy module for determining a diagnostic value for selected putative gene or gene product triplets; and

an interrelationship score determination module for determining an interrelationship score for at least some of the selected gene or gene product triplets, wherein the interrelationship score reflects the strength of the gene or gene product triplet interrelationship.

In yet another aspect of the invention, a machine-readable medium having recorded thereon machine-readable information is provided. In one embodiment of the invention, the machine-readable information can cause a computer to:

receive input data obtained from gene expression profiling, wherein the input data represents a direction and a magnitude of expression of profiled genes or gene products;

transform the input data to thereby map the input data onto a three-dimensional response surface and generate putative gene or gene product triplets of interrelated genes or gene products, wherein the three-dimensional response surface describes a regulatory interrelationship among the profiled genes or gene products;

evaluate the transformed data for each putative gene or gene product triplet to determine a lack-of-fit value therefore;

evaluate the lack-of-fit value for selected putative gene or gene product triplets to determine a diagnostic value therefore; and

evaluate the lack-of-fit value and the diagnostic value for at least some of the selected putative gene or gene product triplets to determine an interrelationship score therefore, wherein the interrelationship score reflects the strength of the gene or gene product triplet interrelationship.

BRIEF DESCRIPTION OF THE DRAWINGS

Figure 1 illustrates a data processing flowchart of a method according to one embodiment of the invention.

Figure 2 illustrates a three-dimensional response surface according to one embodiment of the invention.

Figure 3 illustrates an exemplary activator-repressor-target biological model according to one embodiment of the invention.

Figure 4 illustrates a predicted gene regulatory network generated using yeast GeneChip® data and known biological knowledge according to one embodiment of the invention.

Figure 5 illustrates a predicted gene regulatory network generated using yeast microarray data.

Figure 6 illustrates a system for managing and or analyzing gene expression data using an SRS algorithm according to one embodiment of the invention.

DEFINITIONS

For purposes of clarification, and to facilitate an understanding of the present invention and the embodiments disclosed herein, a number of terms used herein are defined as follows.

<u>Gene Expression Profiling</u>: A process by which molecular techniques are used to measure and compare expression levels of certain nucleic acid sequences (e.g., mRNAs, genes, expressed sequence tags (ESTs)), or levels of certain gene products, such as amino acid sequences (protein or fragments of proteins), in a cell-derived sample in relation to the levels of the same nucleic acid sequences or gene products from a different sample, or from the same sample measured at a different time point.

<u>Gene</u>: A sequence of nucleotides specifying a particular polypeptide chain.

<u>Gene product</u>: One or several polypeptide chains of amino acids translated from RNA transcribed from a gene.

<u>Activator/Repressor</u>: Proteins, such as transcription factors and tumor suppressors, capable of inducing physiological change, usually enhancement or inhibition of gene expression in a given biological system (activation or deactivation), typically in response to an environmental stress.

<u>mRNA</u>: Messenger Ribonucleic Acid.

DETAILED DESCRIPTION OF THE INVENTION

The present invention provides a method and system for managing and analyzing gene expression data using a Smooth Response Surface (SRS) algorithm. Generally, response surface methodologies focus on the relationship between input factors and response in an evaluation of a process or system (Wu and Hamada, *Experiments: Planning, Analysis and Parameter Design Optimization*, New York: Wiley, 2000).

In one embodiment of the invention, the SRS algorithm provides a biological model which describes the qualitative relationship between a target gene or gene product, a repressor gene or gene product, and an activator gene or gene product. Such an SRS algorithm can efficiently extract information from gene expression profiling data to bear on the activator-repressor-target model. More particularly, the SRS algorithm can use a three-dimensional response surface as a graphic representation of a high-dimensional decision matrix (ie., n by n decision matrix as n tends to infinity), which leads to a direct process of transforming quantitative expression data.

In contrast, the fuzzy logic method of the prior art uses heuristic rules in a decision matrix, and consists of a stepwise process of fuzzification, decision-making, and defuzzification. Some advantages of analyzing gene expression data using an SRS algorithm rather than a fuzzy logic approach can include noise tolerance, computation efficiency, and simpler data processing, e.g., from stepwise process to a direct transformation.

More particularly, compared with the fuzzy logic approach of the prior art, there are many advantages in analyzing gene expression data with the SRS algorithm. For instance, the SRS algorithm is more noise tolerant. The SRS algorithm is less sensitive to noise because of its smooth response surface, while the fuzzy logic approach is more sensitive to noise because of its discrete decisions (therefore discontinuous response surface). For example, given genes $A = 0.10$ and $B = 0.49$, if the noise causes gene $B' = 0.51$, then in the SRS algorithm the fitted values of the product genes are given by $C = S(A,B) = 0.11$ and $C' = S(A, B') = 0.098$, which results in the noise-induced-error $C - C' = 0.012$. In contrast, in the fuzzy logic method, $C = 0.305$ and $C' = 0.1475$, so the noise-induced-error is given by $C - C' = 0.1575$.

This example shows the noise-induced-error in the fuzzy logic approach is more serious than that in the SRS algorithm. Thus, in the fuzzy logic approach, noise levels can result in informational loss. In other words, the SRS makes more accurate decisions with noise-induced error than the fuzzy logic approach does. Moreover, the SRS algorithm predicts a larger and more complicated gene regulatory network than the fuzzy logic approach for the same GeneChip® expression data. Additionally, the SRS algorithm is significantly more efficient in computation because of mathematical simplicity and compactness. It has shown tremendous saving in computing time. For instance, the fuzzy logic approach takes about 200 hours on an 8-processor SGI origin 2000 to analyze the relationships between 1,898 genes for GeneChip® yeast data. In contrast, the SRS algorithm only takes about 4 hours.

In a particular embodiment of the invention, the SRS algorithm can be applied to known expression data sets, such as yeast expression data gathered from the Affymetrix GeneChip® system and/or a cDNA microarray. By using gene expression profiling data collected at different time points of the cell cycle, the SRS algorithm can predict and/or identify many regulatory elements and their target genes or gene products within the cell which work together to maintain and control certain cellular processes.

Many gene or gene product interrelations predicted and/or identified by the SRS algorithm can be independently verified and validated by available experimental results. For example, when analyzing yeast gene expression data, the predicted signaling network controlling anaerobic and aerobic growth and cell proliferation can be verified with known experiment evidence. Such results verify that the SRS algorithm can accurately identify and predict biologically relevant connections between sets of genes or gene products, which in turn can be used to model the complex web of interactions that regulate gene expression.

The SRS algorithm provides a powerful data mining tool for analyzing gene expression profiling data. In general, the findings of the algorithm agree well with published experimental results from the literature. This should not come as a surprise, seeing as the algorithm searches for relationships that fit our logical understanding of how an activator, repressor, and target should interact. By using essentially the same criteria that an experimenter would use to describe the regulatory function of a protein, the SRS algorithm approximates the thought process an expert would use in analyzing this data. However, by applying a computational algorithm to the analysis of the data, the SRS algorithm provided a "lens" through which the data can be sorted in an unbiased manner, quickly and efficiently.

Although the validation of the SRS algorithm described herein was performed using GeneChip® and microarray data, the SRS algorithm can be used equally well with any other expression profiling technique known in the art, such as but not limited to Sequential Analysis of Gene Expression (SAGE). SAGE has the advantage that it can detect completely unknown proteins, while GeneChip® technologies require that at least the sequence of a protein's mRNA be known. This ability to detect unknown proteins is particularly well suited to the functional characterization that the SRS algorithm makes possible.

Similarly, although generally described as being used to search for triplets of activator, repressor, and target genes or gene products, the SRS algorithm is not so limited and can be applied to other relationships and more complicated systems. Examples include, but are not limited to, other classes of relationships such as co-activators and co-repressors, or more complicated systems that involve genes whose transcription is regulated in complex ways by any number of transcription factors.

One application of the SRS algorithm is to independently validate or discover drug targets. Traditional techniques for drug target discovery require a detailed understanding of the biology underlying the disease, which can be slow and difficult to obtain. In contrast, expression profiling is a rapid high-throughput process, providing a large amount of information about the cell in a form that can be easily processed on a computer. By using a SRS approach to analyzing expression profiling data, it is possible to confirm the mechanism of a known target. Moreover, because the SRS algorithm does not require biological information about the gene, genes encoding proteins with unknown functions can be included just as easily as genes encoding proteins with known functions. This ability to identify functional clues for uncharacterized genes is a great advantage in drug target discovery.

*Method for Analyzing Gene Expression Data Using an SRS Model*

Generally, in one aspect of the invention, a method for managing and analyzing information obtained from differential expression of genetic information in biological cells is provided. In one embodiment, the method comprises receiving input data obtained from gene expression profiling, wherein the input data represents a direction and a magnitude of the expression of profiled genes or gene products. The input data is then transformed to thereby map the input data onto a three-dimensional response surface and generate putative gene or gene product triplets of interrelated genes or gene products, wherein the three-dimensional response surface describes the regulatory interrelationship among the profiled genes or gene products.

Once the input data is transformed and putative gene or gene product triplets are generated, the transformed data for each putative gene or gene product triplet is evaluated to determine a lack-of-fit value therefore. The lack-of-fit value for selected putative gene or gene product triplets is then evaluated to determine a diagnostic value therefore.

All of the putative gene or gene product triplets may be selected for evaluation by the diagnostic strategy, or alternatively, only a portion of the putative gene or gene product triplets may be selected for further evaluation. In one embodiment, the putative gene or gene product triplets for further evaluation may be selected based on a predetermined lack-of-fit value cutoff. Once the lack-of-fit and diagnostic values are determined, at least some of the selected putative gene or gene product triplets may be further evaluated to determine a interrelationship score therefore, wherein the interrelationship score reflects the strength of the gene or gene product triplet interrelationship.

Again, all of the putative gene or gene product triplets evaluated by the diagnostic strategy may be further processed to determine their interrelationship score. Alternatively, interrelationship scores may only be determined for a portion of the putative gene or gene product triplets evaluated by the diagnostic strategy. In one embodiment, the putative gene or gene product triplets processed to determine their interrelationship score may be selected based on a predetermined diagnostic value cutoff.

In one embodiment of the invention, once the interrelationship scores are determined, a gene or gene product regulatory network may be constructed based upon the gene or gene product triplet scores, and optionally, known biologically functions and relationships.

The method may also include a data filtering step wherein the input data is filtered to ensure the input data are above a predetermined noise level. In one embodiment, such input data filtering can involve only accepting input data with a maximum fold-change of at least about 3 and a maximum intensity of at least about 100. Further, a data imputation step may optionally be employed to replace missing, negative, and/or small positive values in the input data. For instance the imputing can involve replacing about the lowest 5% of input data values with values representing about the fifth percentile of all genes included in the input data for each time point of the input data.

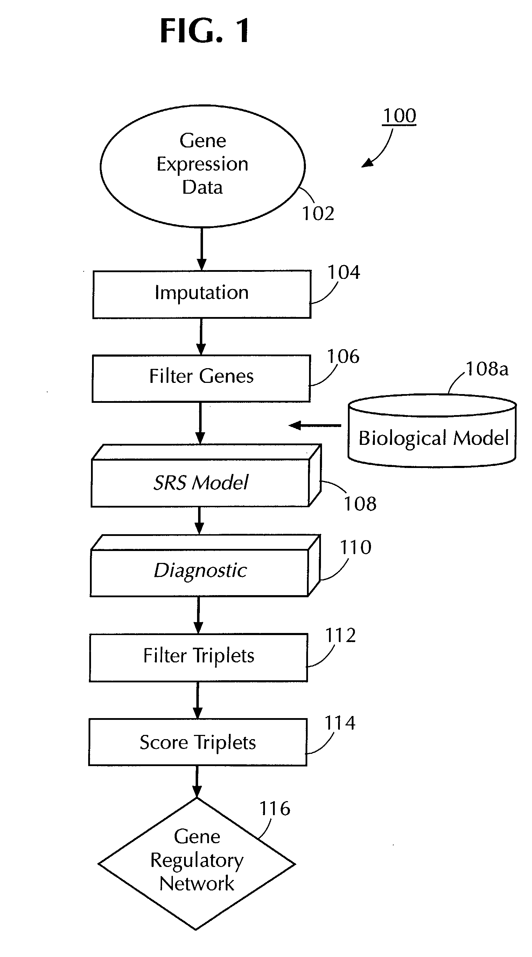

With reference to Figure 1, an SRS algorithm 100 according to one embodiment of the invention is illustrated. As shown, initially, gene expression profiling data is generated, obtained, and/or received 102 from an expression profiling data source. The optional imputation 104 and gene filtering 106 steps may then be applied for removing noise from the data to ensure that the expression data is above a predetermined noise level and the observed signal change in the data is significant. The input data, optionally imputed and filtered, is then transformed using an SRS model 108 to map the data onto a three-dimensional response surface and thereby generate putative gene and/or gene product triplets. Such a response surface may be defined as a function of a pair of genes, which builds a relationship between the target and activator-repressor.

For each putative triplet of genes and/or gene products *(A,B,C),* a lack-of-fit formula is defined using biological model information to determine the closeness of fit of the transformed data values to observed biological model values 108a. A diagnostic strategy 110 is then applied to selected putative triplets, and the selected putative triplets may optionally be further filtered 112 to refine the putative triplet sample. An interrelationship score 114, which reflects the strength of the triplet interrelationship, is then determined to rank the refined putative triplets, and a gene regulatory network 116 may be constructed from the interrelationship scores. Optionally, the gene regulatory network may be verified using known biological functions and relationships (not shown).

The gene expression profiling input data may be generated, provided, obtained, or received in any manner known in the art, including but not limited to GeneChip® and microarray techniques. By way of example, a sample of RNA may be monitored using an expression profiling array, such as an Affymetrix GeneChip® probe array having, for example, oligonucleotides corresponding to the human genome, capable of detecting expression levels for over 6,000 sequences for that genome. Affymetrix provides a GeneChip® fluidics station that automates the hybridization of nucleic acid targets to a probe array cartridge, and thus controls the delivery of reagents and the timing and temperature for hybridization. Each fluidics station can independently process four probe arrays at a given time.

Accordingly, each target may be prepared from a set of cell dishes by isolation of RNA over a course of time. The treatment of those cells may be emulated by adding, for example, serum thereto. At predetermined intervals, a small amount of the fluid is removed, and the cells are put in a quiescent state to stop the reaction time. Accordingly, a large set of targets, having a predetermined amount of liquid (e.g., 0.5 mL each) is produced. The GeneChip® fluidics station will then automatically hybridize each target, i.e., it will extract all the RNA and label the RNA by adding a chemical tag to each molecule, and control the delivery of the resulting liquid to the probe arrays to facilitate obtaining sequencing information regarding the mRNAs.

This is done by the probe arrays exposing the target to light at a predetermined location and measuring the photons collected at various locations within the arrays. The amount of mRNA is then ascertained based upon the signal strength of the reading given by the probe at the appropriate location corresponding to that sequence or sequence segment. A net change in signal may indicate activation or repression of gene transcription, or possibly post-transcriptional stabilization the mRNA due a given set of treatment conditions.

The expression data so obtained may then optionally be imputed and filtered to create suitable sets of data for further analysis, essentially as diagrammed in Figure 1 and described herein. Such optional filtering can serve to ensure selected data are above a minimum noise threshold and represent gene whose net change in expression satisfies a predetermined level of signal strength.

The SRS algorithm may also be used to analyze data sets generated by translated products of genes expressed in biological cells under control and treatment conditions. For example, the ProteinChip™ System (Ciphergen Biosystems, Palo Alto, CA) consists of microchips having activated surfaces that allow immobilization of antibodies, receptors, or DNA for capturing proteins from a sample. After sample binding, the array is washed to remove unbound molecules. Thereafter, energy adsorbing molecules are added to facilitate detection captured proteins via, for example, mass spectrometry. Multiple sets of protein expression spectra may be obtained and filtered to create suitable sets of data for analysis according to the illustrated embodiments of the invention.

*System for Analyzing Gene Expression Data Using an SRS Model*

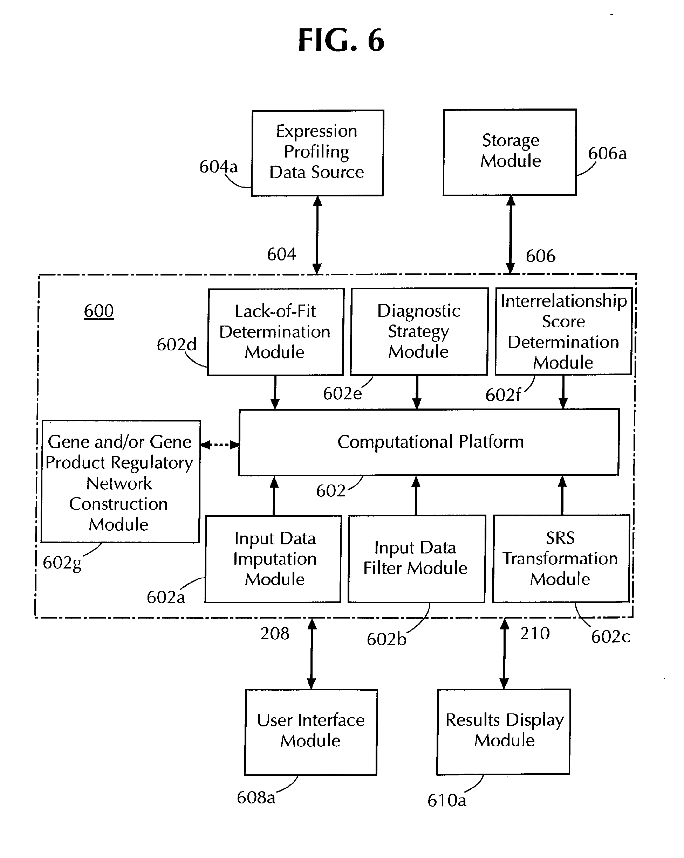

In another aspect of the invention, a system for managing and/or analyzing gene expression data using an SRS algorithm is provided. With reference to Figure 6, a system according to one embodiment of the invention is illustrated. As shown, a system 600 according to the illustrated embodiment of the invention generally includes a computational platform 602 including an SRS data transformation module 602c for mapping input data onto a three-dimensional surface and generating putative gene or gene product triplets of interrelated genes or gene products, wherein the three-dimensional response surface describes the regulatory interrelationship among the profiled genes or gene products.

The illustrated embodiment further includes a first interface 604 to a expression profiling input data source, wherein the input data represents a direction and a magnitude of expression of profiled genes or gene products. Optionally, a second interface 606 to a storage module 606a, a third interface 608 to a user interface module 608a, and/or a fourth interface 610 to a results display module 610a can also be included.

The expression profiling data source may be integrated with the user interface to provide for automated information retrieval, manual user information input, or a combination thereof. Further, the computational platform, the expression profiling data source, the results display module, and/or the storage module may be integrated into a single standalone computer, may be distributed over several computers, or may be networked together via intranet or internet.

The optional storage module can interface with the computational platform, the expression profiling data source, and/or the user interface to store information generated or provided by the computational platform, expression profiling data source, and/or the user interface for subsequence retrieval, analysis, use, and/or display. Among other things, the results display module can display results from the computational platform and/or information from the expression profiling data source, and can include a printer or monitor, or can employ email or other data file output (e. g., audio, video, graphical, or textual).

The computational platform 602 of the illustrated embodiment of the invention may further include an input data imputation module 602a for imputing missing, negative, and/or small positive input data values, and an input data filtering 602b for filtering the input data below a noise threshold. The computational platform 602 may also include a lack-of-fit determination module 602d, a diagnostic strategy module 602e, and an interrelationship score module 602f. In one embodiment of the invention, the computational platform 602 can further include a gene or gene product regulatory network construction module 602g.

In one embodiment of the invention, the expression profiling source 604a may comprise, for example, a cDNA microarray or GeneChip® which generates input data representing a direction and a magnitude of regulation of a gene or gene product. The expression profiling source may also include a data storage module (not shown) for storing that expression profiling data in an organized fashion in a database. Alternatively, the expression profiling source 604a may be a database which includes expression profiling data previously generated.

The various modules of the computational platform may be integrated into a single standalone computer, may be distributed between computers, or may be networked via intranet or internet. Further, a system of the invention may be implemented by hardware or a combination of hardware and software. The software may be recorded on a medium for reading into a computer memory and executing. The medium may be, but is not limited to, for example, one or more of a floppy disk, a CD ROM, a writable CD, a Read-Only-Memory (ROM), and an Electrically Alterable Programmable Read Only Memory (EAPROM).

*SRS Model Development*

In developing the SRS algorithm, an activator-repressor-target biological model was employed to search for triplets of genes or gene products *(A,B,C)* under which, when the activator *A* is high and the repressor *B* is low, the concentration of the target gene *C* is high. Conversely, when the concentration of the repressor *B* is high and the activator *A* is low, the concentration of the target *C* is low. These qualitative, or heuristic, rules can be used in combination with compact and explicit mathematical formulae to extend a decision matrix, such as the matrix associated with a fuzzy logic algorithm, to a high-dimensional decision matrix associated with a SRS algorithm of the invention.

Thus, in one embodiment of the invention, gene expression profile input data parameters are designated as follows, wherein $a_n$, $b_n$, and $c_n$ represent respective gene and/or gene product concentrations at time $n$.

$$A = (a_1,...,a_n)$$

$$B = (b_1,...,b_n)$$

$$C = (c_1,...,c_n)$$

Response Surface Function

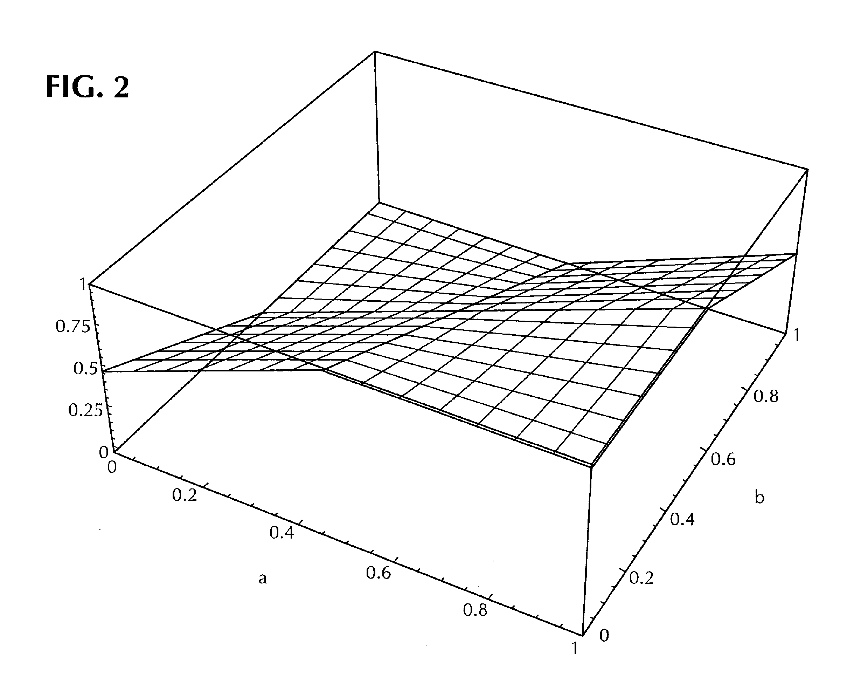

In one embodiment of the invention, to transform the gene profile expression data and fit it onto a response surface model, the raw data over the time points may first be normalized into the interval [0,1] such that the minimum and maximum values are 0 and 1 respectively. Next, a three-dimensional smooth response surface given by *S(A,B)*, is defined as a piecewise linear-quadratic polynomial on $[0,1] \times [0,1]$. Such a polynomial generally describes a surface in a three-dimensional unit cell as shown in Figure 2, and can be used to describe the relationship between the target and activator-repressor in the biological model.

The three-dimensional response surface may be interpreted as a graphic representation of a high dimensional decision matrix. After normalization, the normalized values *A* and *B* may be broken into various classes, roughly say, e. g., from "LOW" (if it is close to 0) to "HIGH" (if it is close to 1) or in the between (if it is around 0.5). The function *S (A, B)* maps two normalized values onto the three-dimensional surface to provide a mathematical method of heuristic rules that facilitate decision-making. As seen in Figure 2, a triplet *(A,B, S(A,B))* represents a fitted, i.e., modeled, biological relationship that follows the pattern of a fitted gene product, *S(A,B)*, controlled by both an activator *A* and repressor *B*, as described in a activator-repressor-target model. The response surface captures a biological model with features such as compactness, simplicity, and visualization.

In one embodiment of the invention, the expression data can be transformed according to equation set 1.

$$S(a,b) = \begin{cases} 2a(1-b), & \text{if } 0 \le a \le 0.5 \ \text{and} \ 0.5 \le b \le 1; \\ 1 - 2(1-a)b, & \text{if } 0.5 \le a \le 1 \ \text{and} \ 0 \le b \le 0.5; \\ a - b + 0.5, & \text{otherwise} \end{cases} \quad (1)$$

Lack-of-fit Function

Generally, a lack-of-fit value describes how well the fitted, i.e., modeled, putative triplet values fit the observed biological model. Accordingly, for each putative gene or gene product triplet *(A,B,C)*, the fitted value of a gene or gene product *C* may be given by $C = S(A, B)$, and the actual observed value may be obtained from the expression profiling data set. The lack-of-fit formula, defined as *RT(A,B,C)* represents the ratio of the residual sum of squares and the total sum of squares.. The smaller the lack-of-fit value, the better the response surface captures the observed biological relationship. In one embodiment of the invention, to save storage and computation time, only those triplets with the lack-of-fit value not greater than a predetermined *RT* value are kept for further consideration.

In one embodiment of the invention, the *RT (A,B,C)* may be calculated according to equation 2.

$$RT(A,B,C) = \frac{\sum_{i=1}^{n}(c_i - \hat{c}_i)^2}{\sum_{i=1}^{n}(c_i - \overline{c})^2} \quad (2)$$

where

$$\overline{c} = \frac{1}{n}\sum_{i=1}^{n}c_i$$

is the average of the actual observed values.

Diagnostic Function

After any initial filtering, a diagnostic strategy is applied to check the reliability of selected putative triplets. For each putative triplet *(A,B,C),* a summary diagnostic measure can be given by *Diag(A,B,C)* which is a measure of robustness of the model fitting. It is observed that if the intensity measurement at one or two time points significantly deviate from the model, such a deviation may suggest that the measurement is faulty and should be treated as an outlier. For instance, if such a deviation occurs at the $i^{th}$ time point, then $RT_{(i)}(A,B,C)$, i.e., the lack-of-fit of *(A,B,C)* when the $i^{th}$ time point (or say, the $i^{th}$ column) is left out, will differ greatly from *RT (A,B,C),* so as to cause some justification in the diagnostic measure formula.

Instead of doing this diagnostic check for each time point, *Diag (A,B,C)* can provide a summary measure over all time points for a given putative triplet, therefore to detect false triplets. A larger diagnostic measure generally suggests that the information for the triplet may be unreliable and the triplet may then be removed from further consideration. As such, in one embodiment of the invention, the criteria for selecting putative triplet candidates can be as follows: *RT (A,B,C)* ≤*RT* and *Diag (A,B,C)* ≤*Diag, where RT* and *Diag* are predetermined values, as specified by a user.

In one embodiment of the invention, the value for *Diag (A,B,C)* may be determined using equation 3.

$$Diag(A,B,C) = \frac{\left(\frac{1}{n}\sum_{i=1}^{n}\left[RT_{(i)}(A,B,C) - RT(A,B,C)\right]^2\right)^{\frac{1}{2}}}{RT(A,B,C)} \qquad (3)$$

The diagnostic strategy can serve to ensure that selected putative gene and/or gene product triplets with unreliable measurements at some time points are properly filtered without losing the informational content they include. By way of example, for the yeast GeneChip® data described herein, there is one extreme time point (the 90-minute) whose scale is quite different from others. One prior art filtering strategy is to remove the whole column from the data (e.g., Tamayo et al., *PNAS*, 1999;96:2907-2945). Removing the whole column can result in a severe loss of information as the extreme values only occur for some genes. However, for other genes in the same column, the information they carry is valuable to expression analysis. Thus, the leave-one-out diagnostic strategy of the SRS algorithm has the ability to extract useful information from that time point, while minimizing error from the noise.

Interrelationship Score Function

Finally, an interrelationship score, which reflects the strength of the putative triplet interrelationship, *Score (A, B,C)*, may be determined for each chosen putative triplet. Generally, the interrelationship score may be a function of the lack-of-fit and the diagnostic measure values. For instance, the interrelationship score function may be determined primarily based on the *RT(A,B,C)* value and secondarily based on the *Diag(A,B,C)* values. As such, triplets with lower values of *RT(A,B,C)* and *Diag(A,B,C)* have lower interrelationship scores. The lower value the *Score(A,B,C),* the closer the relationship among the putative triplet *(A,B,C).*

In one embodiment of the invention, the interrelationship score may be determined according to equation 4.

$$Score(A,B,C) = RT(A,B,C)(1 + Diag(A, B, C)) \qquad (4)$$

Certain implementations or embodiments of the invention are further illustrated in the following, non-limiting example.

EXAMPLES

Yeast Genechip Data

The SRS algorithm was applied to a public oligonucleotide GeneChip® gene expression profiling data describing the yeast cell cycle (Cho et al., *Molecular Cell*, 1998;2:65-73). The data were collected at 17 time points for 6457 genes (including 45 unlabeled genes). First, negative and small positive values, which are due to measurement error and thus not reliable, were imputed (i.e., replaced). The lowest 5% of the data values were replaced by the fifth percentile over all genes profiled for each time point. The fifth percentiles generally varied from tens to twenties over the 17 time points.

Next, the profiled gene data was filtered such that the maximum fold-change (i.e., ratio of the maximum and minimum values) was at least about 3 and maximum intensity was at least about 100. After filtering, 1514 genes were retained and these genes were transformed by the SRS algorithm to form a triplet candidates pool. After transformation, there were 28,023 triplets, out of $1514 \times 1513 \times 1512 \approx 3.5 \times 10^9$ triplets, whose lack-of-fit values were at most about 0.1. These triplets were then further filtered by the diagnostic strategy to result in 20,500 triplets having a diagnostic value of at most about 2.0. Finally, the score function was used to generate a score value which ranked these total 20,500 triplets.

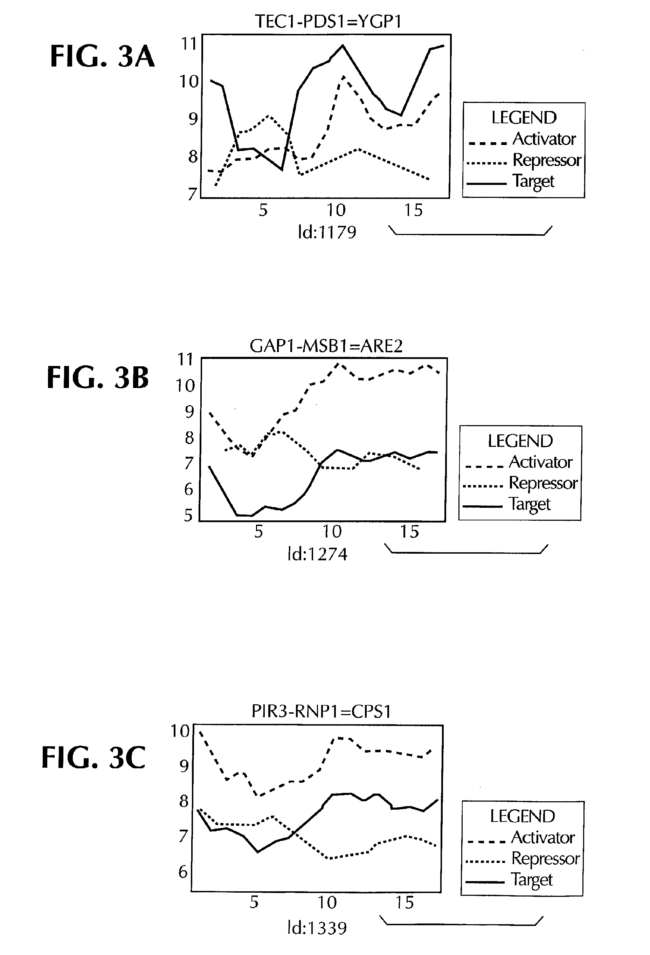

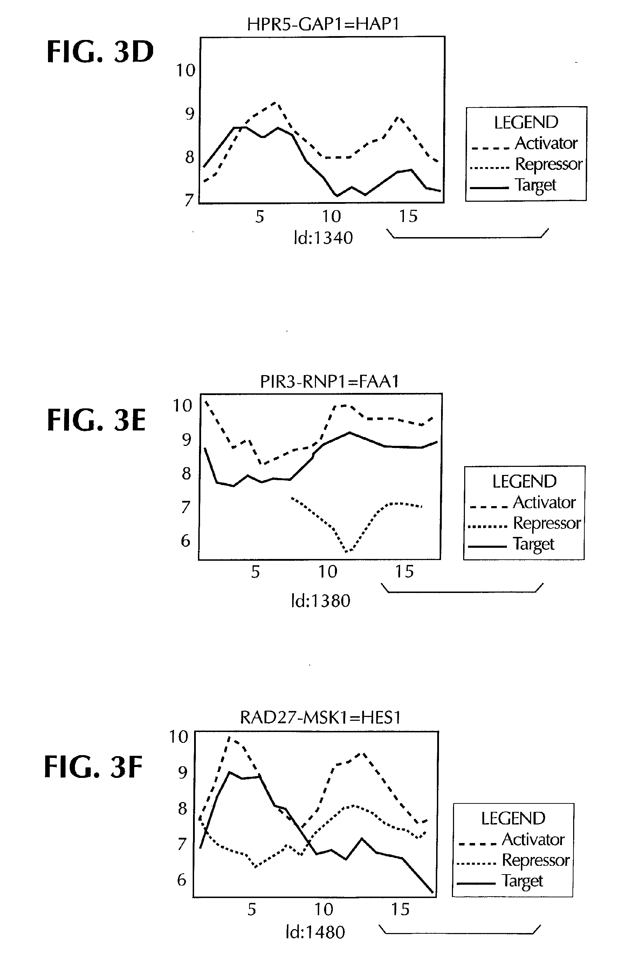

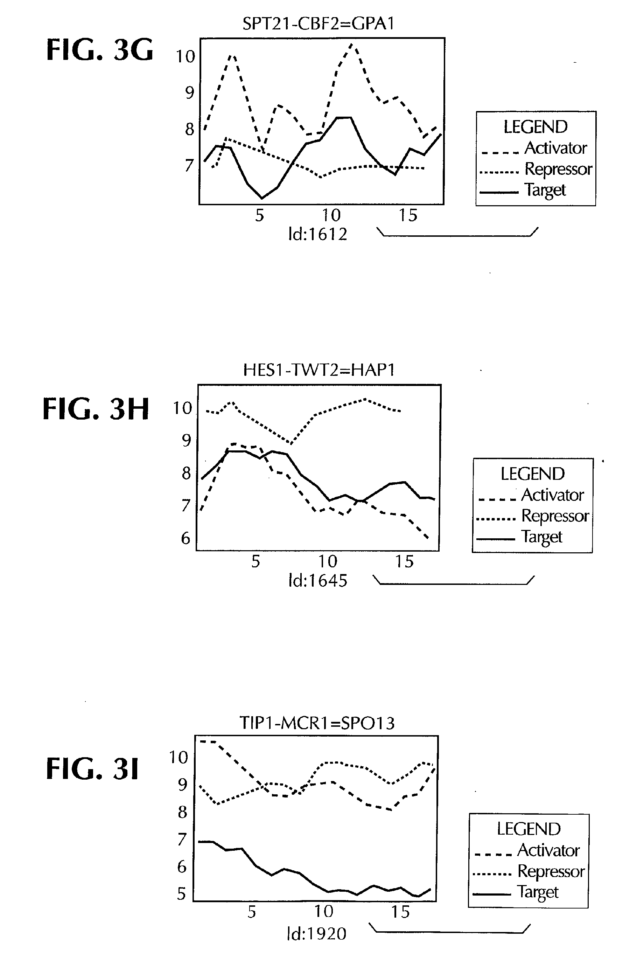

Table 1 lists the top scoring triplets with known functions and Figure 3 shows the biological model for the first nine triplets. More particularly, Figure 3 illustrates graphs showing the log transformed (base 2) concentration values of the first nine triplets. The graphs capture the biological model, i.e., the observed biological relationship among the triplets. For instance, when the concentration of the activator A (long-dashed line) is high and that of the repressor B (short-dashed line) is low, the concentration of the target C (solid line) is high. Likewise, when the concentration of the activator A is low and the concentration of the repressor B is high, the concentration of the target C is low.

Table 1.

| Top Scoring Triplets With Known Functions From the Yeast Genechip Data | | | | | | |
|---|---|---|---|---|---|---|
| Rank | A | B | C | RT (A, B, C) | Diag (A, B, C) | Score (A, B, C) |
| 1179 | TEC1 | PDS1 | YGP1 | 0.0553 | 0.48307 | 0.08201 |
| 1274 | GAP1 | MSB1 | ARE2 | 0.06608 | 0.25991 | 0.08325 |
| 1339 | PIR3 | RNP1 | CPS1 | 0.07207 | 0.16708 | 0.08411 |
| 1340 | HPR5 | GAP1 | HAP1 | 0.07227 | 0.16418 | 0.08414 |
| 1380 | PIR3 | RNP1 | FAA1 | 0.07604 | 0.1136 | 0.08468 |
| 1480 | RAD27 | MSK1 | HES1 | 0.06075 | 0.41396 | 0.08589 |
| 1612 | SPT21 | CBF2 | GPA1 | 0.07278 | 0.19939 | 0.0873 |
| 1645 | HES1 | TWT2 | HAP1 | 0.06965 | 0.25932 | 0.08771 |
| 1920 | TIP1 | MCR1 | SPO13 | 0.07994 | 0.13201 | 0.0905 |
| 1947 | HPR5 | PEPS | HAP1 | 0.08003 | 0.13536 | 0.09086 |
| 2117 | TIP1 | AGP1 | SPO13 | 0.08185 | 0.12926 | 0.09243 |
| 2226 | INH1 | CBF2 | YGP1 | 0.07592 | 0.22926 | 0.09333 |
| 2243 | CYB2 | CIK1 | TIP1 | 0.07185 | 0.30092 | 0.09347 |
| 2277 | RAD27 | CPS 1 | HES1 | 0.06435 | 0.45645 | 0.09373 |
| 2503 | CYB2 | MCR1 | SPO13 | 0.06806 | 0.40383 | 0.09555 |
| 3107 | HES1 | GPD2 | HAP1 | 0.08957 | 0.12041 | 0.10036 |
| 3122 | KAR3 | FAA1 | HAP1 | 0.08402 | 0.19595 | 0.10048 |
| 3186 | INH1 | PDS1 | YGP1 | 0.08022 | 0.25682 | 0.10082 |
| 3941 | IPL1 | TSM1 | CBF2 | 0.09555 | 0.09849 | 0.10497 |
| 4273 | SPO16 | ASE1 | SMC3 | 0.09026 | 0.18177 | 0.10666 |
| 4437 | TEC1 | HES1 | ARE2 | 0.09672 | 0.11106 | 0.10746 |
| 4458 | CDC17 | MYO3 | CDC21 | 0.08964 | 0.20019 | 0.10759 |
| 4547 | MEP2 | MSB1 | ARE2 | 0.0856 | 0.26126 | 0.10797 |
| 4646 | GLK1 | RNP1 | FAA1 | 0.09279 | 0.16874 | 0.10845 |
| 4697 | CYT1 | HAP1 | ARE2 | 0.09521 | 0.14227 | 0.10875 |
| 4764 | CYB2 | HAP1 | CYC7 | 0.08143 | 0.3403 | 0.10914 |
| 4941 | TIP1 | PEP5 | SPO13 | 0.09483 | 0.15952 | 0.10996 |
| 5052 | TIP1 | GAP1 | SPO13 | 0.09567 | 0.15466 | 0.11046 |
| 5160 | SRO4 | MYO3 | CDC21 | 0.09807 | 0.13158 | 0.11097 |

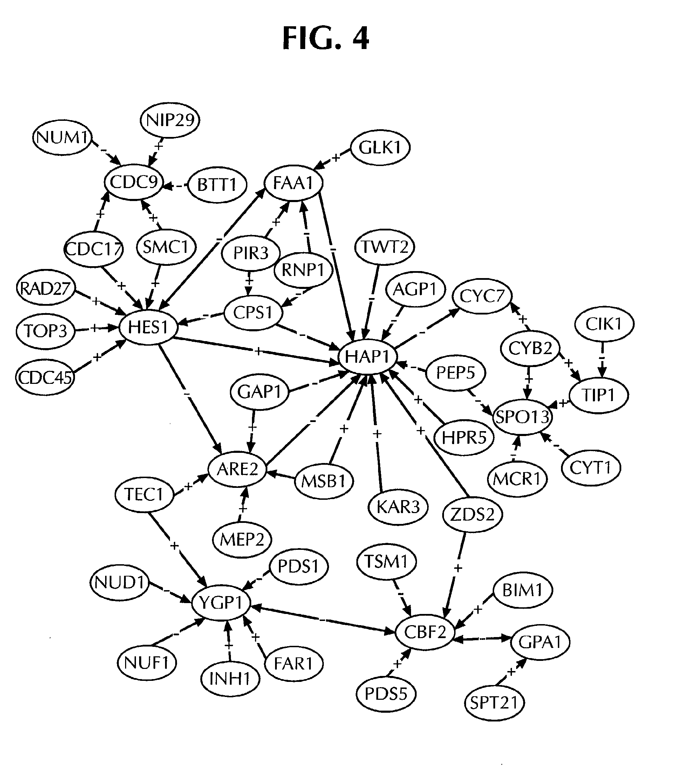

In order to evaluate and validate the algorithm, the best scoring triplets were examined to see if they made biological sense. Figure 4 shows a connected network of all triplets that have known functions. Within this network, many predicted activator-repressor-target relationships have been confirmed by published experimental results. Looking at a few of common targets in this network, most associated regulatory genes either carry similar cellular functions or are involved in a same cellular process. For example, CDC9 encodes an ATP-dependent DNA ligase and is an essential gene for cell division and DNA recombination. Further, four of the identified regulators are functionally related. SMC1 acts as a positive regulator and is a chromosomal ATPase family member. Like CDC9, SMC1 is involved in chromosome structure and segregation. Another positive regulator is NIP29, which is a structural protein associated with microtubule nucleation and spindle body duplication. Of the two identified negative regulators, BTT1 has repressor effects on expression of several genes and NUM1 functions in nuclear migration and microtubule polymerization.

Another major node in the predicted network highlights HAP1. The transcription factor HAP1 has been shown to repress the nuclear encoding cytochrome gene CYC7 under anaerobic growth and to activate CYC7 under aerobic growth. The prediction suggests that HAP1 repress CYC7, which in turn accurately predicts that the cells used in the data set were primarily grown under anaerobic conditions. Two other gene products, FAA1 and HES1, which were predicted to be involved in cellular lipid metabolism and ergosterol biosynthesis, have also been implicated in HAP1 regulation in literature information.

In addition, the SRS algorithm uncovered relationships for SPO13, CBF2 and YGP1. SPO13 acts as a transcriptional activator and controls meiotic chromosome segregation. CBF2 is a centromere-binding factor in a multisubunit kinetochore protein complex. YGP1 codes for a glycoprotein synthesized in response to nutrient limitation. The common theme in these gene products is their functions in cell proliferation and cell division. As expected, many genes associated with each of them have been shown to carry similar functions.

*Yeast Microarray Data*

The SRS algorithm was also applied to a yeast cDNA microarray gene expression profiling data, which were collected at 15 time points for 2,467 genes (cdc15 of Eisen et al., *PNAS,* 1998;95:14863-14868). The Cy5/Cy3 fluorescence ratios were used for analysis. First, any missing profiling values were imputed by replacing the missing value with the average of its previous and following time points. Such a replacement was done for each profiled gene. Each profiled gene was removed if it had two or more consecutive missing time points. After the imputation, each profiled gene was filtered if its maximum fold-change was less than about 3. After filtration, 830 genes were retained and transformed to fit the SRS model.

There were 2,393 triplets whose lack-of-fit were at most about 0.1 and diagnostic measure were at most about 2.0. These 2,393 triplets were then scored for further investigation.

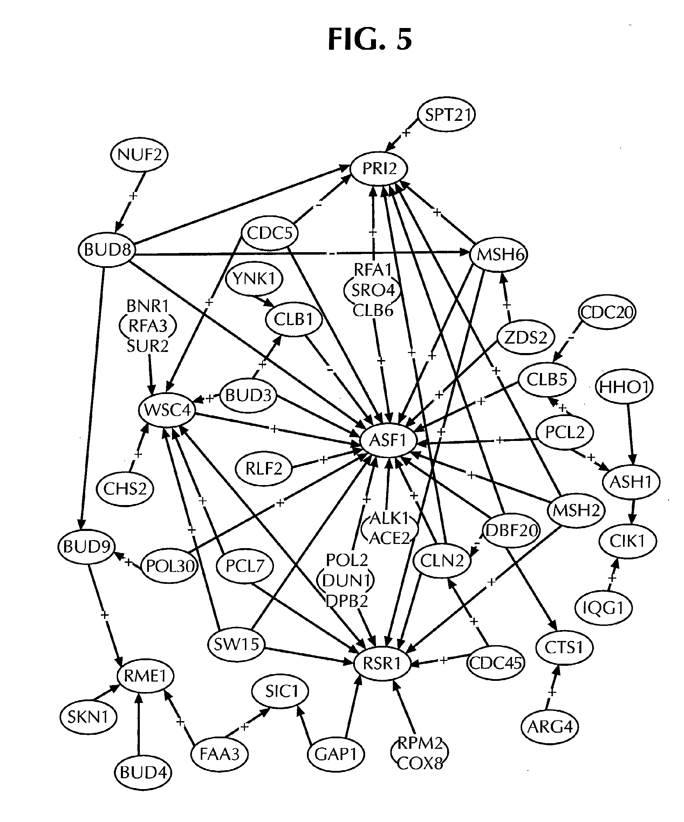

In order to evaluate and validate the results predicted by SRS algorithm, the best scoring triplets that have known functions were examined again. Within the network illustrated in Figure 5, many predicted activator-repressor-target relationships have been confirmed by published experimental results. For example, RSR1 is a RAS GTPase involved in bud site selection. The predicted RSR regulators include CDC45, POL2, DPB2, SWI5 and RPM2. These proteins also are known function in budding and cell proliferation. Additionally, ASF1 causes depression of many silent chromosomal loci when overexpressed in a cell. Because of its broad functionality, a large number of triplets have been found with ASF1 included. Furthermore, ASF1 associated proteins have diverse cellular roles.

While the invention has been described by way of example embodiments, it is understood that the words which have been used herein are words of description, rather than words of limitation. Changes may be made, within the purview of the appended claims, without departing from the scope and spirit of the invention in its broader aspects. Although the invention has been described herein with reference to particular means, materials, and embodiments, it is understood that the invention is not limited to the particulars disclosed. The invention extends to all equivalent structures, means, and uses which are within the scope of the appended claims.

**Claims**

1. A method for managing and analyzing information obtained from differential expression of genetic information in biological cells, the method comprising:

   receiving input data obtained from gene expression profiling, wherein the input data represents a direction and a magnitude of expression of profiled genes or gene products;
   transforming the input data to thereby map the input data onto a three-dimensional response surface and generate putative gene or gene product triplets of interrelated genes or gene products, wherein the three-dimensional response surface describes a regulatory interrelationship among the profiled genes or gene products;
   evaluating the transformed data for each putative gene or gene product triplet to determine a lack-of-fit value therefore;
   evaluating the lack-of-fit value for selected putative gene or gene product triplets to determine a diagnostic value therefore; and
   evaluating the lack-of-fit value and the diagnostic value for at least some of the selected putative gene or gene product triplets to determine an interrelationship score therefore, wherein the interrelationship score reflects the strength of the gene or gene product triplet interrelationship.

2. The method of Claim 1, further comprising
   filtering the input data to ensure the input data are above a predetermined noise level.

3. The method of Claim 2, wherein the filtering comprises:

   only accepting input data with a maximum fold-change of at least about 3 and a maximum intensity of at least about 100.

**4.** The method of Claim 1, further comprising:

imputing the input data to replace missing, negative, and/or small positive values in the input data.

**5.** The method of Claim 4, wherein the imputing comprises:

replacing about the lowest 5% of input data values with values representing about the fifth percentile of all genes included in the input data for each time point of the input data.

**6.** The method of Claim 1, further comprising:

constructing a gene or gene product regulatory network using the determined gene or gene product triplet interrelationship scores

**7.** The method of Claim 6, wherein the constructed gene or gene product regulatory network is at least partially verified using known biological function and/or relationship information.

**8.** The method of Claim 1, wherein the selected putative gene or gene product triplets are selected based on a predetermined lack-of-fit value cutoff; or wherein at least some of the selected putative gene or gene product triplets are based on a predetermined diagnostic value cutoff.

**9.** A system for managing and analyzing information obtained from differential expression of genetic information in biological cells, the system comprising:

a first interface to an expression profiling data source for providing input data obtained from gene or gene product expression profiling to a computational platform, wherein the input data represents a direction and a magnitude of expression of profiled genes or gene products; and
a computational platform comprising:

an SRS transformation module for mapping expression profiling data onto a three-dimensional response surface to generate putative gene or gene product triplets of interrelated genes or gene products, wherein the three-dimensional response surface describes a regulatory interrelationship among the profiled genes or gene products;
a lack-of-fit determination module for determining a lack-of-fit value for each putative gene or gene product triplet;
a diagnostic strategy module for determining a diagnostic value for selected putative gene or gene product triplets; and
an interrelationship score determination module for determining an interrelationship score for at least some of the selected gene or gene product triplets, wherein the interrelationship score reflects the strength of the gene or gene product triplet interrelationship.

**10.** The system of Claim 9 wherein the computational platform is configured in a manner selected from the group consisting of: a stand-alone computer, a distributed computer system, or a networked computer system; or wherein the computation platform further comprises:

an input data filtering module for filtering the input data to ensure the input data are above a predetermined noise level; or wherein the input data filtering module filters the input data by only accepting input data with a maximum fold-change of at least about 3 and a maximum intensity of at least about 100; or wherein the computation platform further comprises:

an input data imputation module for imputing the input data to replace missing, negative, and/or small positive values in the input data; or wherein the imputing module imputes the input data by replacing about the lowest 5% of input data values with values representing about the fifth percentile all genes included in the input data over each time point of the input data; or wherein the computation platform further comprises:

a gene or gene product regulatory network construction module for constructing a gene or gene product regulatory network using the determined gene or gene product triplet interrelationship scores.

11. The system of Claim 10, wherein the computational platform further comprises:

a network verification module for at least partially verifying the constructed gene or gene product regulatory network using known biological function and/or relationship information.

12. The system of Claim 9, wherein the diagnostic strategy module selects the selected putative gene or gene product triplets for diagnostic value determination based on a predetermined lack-of-fit value cutoff; or wherein the interrelationship score determination module selects at least some of the selected putative gene or gene product triplets for interrelationship score determination based on a predetermined diagnostic value cutoff.

13. A machine-readable medium having recorded thereon machine-readable information, such that when the machine-readable information is read and executed by a computer, the machine-readable information causes the computer to:

receive input data obtained from gene expression profiling, wherein the input data represents a direction and a magnitude of expression of profiled genes or gene products;

transform the input data to thereby map the input data onto a three-dimensional response surface and generate putative gene or gene product triplets of interrelated genes or gene products, wherein the three-dimensional response surface describes a regulatory interrelationship among the profiled genes or gene products;

evaluate the transformed data for each putative gene or gene product triplet to determine a lack-of-fit value therefore;

evaluate the lack-of-fit value for selected putative gene or gene product triplets to determine a diagnostic value therefore; and

evaluate the lack-of-fit value and the diagnostic value for at least some of the selected putative gene or gene product triplets to determine an interrelationship score therefore, wherein the interrelationship score reflects the strength of the gene or gene product triplet interrelationship.

14. The machine-readable medium of Claim 13 wherein the machine-readable information further causes the computer to:

construct a gene or gene product regulatory network using the determined gene or gene product triplet interrelationship scores.

15. The method of Claim 14, wherein the machine-readable information further causes the computer to:

at least partially verify the constructed gene or gene product regulatory network using known biological function and/or relationship information.

# FIG. 1

FIG. 2

EP 1 267 295 A1

**FIG. 3A**

TEC1-PDS1=YGP1

LEGEND
- - - Activator
......... Repressor
——— Target

Id:1179

**FIG. 3B**

GAP1-MSB1=ARE2

LEGEND
- - - Activator
......... Repressor
——— Target

Id:1274

**FIG. 3C**

PIR3-RNP1=CPS1

LEGEND
- - - Activator
......... Repressor
——— Target

Id:1339

**FIG. 3D**

HPR5-GAP1=HAP1

Id:1340

LEGEND
- - - Activator
...... Repressor
—— Target

**FIG. 3E**

PIR3-RNP1=FAA1

Id:1380

LEGEND
- - - Activator
...... Repressor
—— Target

**FIG. 3F**

RAD27-MSK1=HES1

Id:1480

LEGEND
- - - Activator
...... Repressor
—— Target

**FIG. 3G**

SPT21-CBF2=GPA1

LEGEND
- - - Activator
........ Repressor
——— Target

Id:1612

**FIG. 3H**

HES1-TWT2=HAP1

LEGEND
- - - Activator
........ Repressor
——— Target

Id:1645

**FIG. 3I**

TIP1-MCR1=SPO13

LEGEND
- - - Activator
........ Repressor
——— Target

Id:1920

# FIG. 4

# FIG. 5

# FIG. 6

Expression Profiling Data Source — 604a

Storage Module — 606a

604

606

600

Lack-of-Fit Determination Module — 602d

Diagnostic Strategy Module

Interrelationship Score Determination Module

602e

602f

Computational Platform

602

Gene and/or Gene Product Regulatory Network Construction Module — 602g

Input Data Imputation Module — 602a

Input Data Filter Module

SRS Transformation Module

208

602b

210

602c

User Interface Module — 608a

Results Display Module — 610a

## EUROPEAN SEARCH REPORT

European Patent Office

Application Number

EP 02 01 1654

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | TAMAYO P ET AL: "INTERPRETING PATTERNS OF GENE EXPRESSION WITH SELF-ORGANIZING MAPS:METHODS AND APPLICATION TO HEMATOPOIETIC DIFFERENTIATION" PROCEEDINGS OF THE NATIONAL ACADEMY OF SCIENCES OF USA, NATIONAL ACADEMY OF SCIENCE. WASHINGTON, US, vol. 96, March 1999 (1999-03), pages 2907-2912, XP000942169 ISSN: 0027-8424 * page 2907, left-hand column, line 1 - line 21 * * page 2907, left-hand column, line 44 - page 2908, left-hand column, line 33 * * page 2908, right-hand column, line 15 - page 2909, left-hand column, line 2 * | 1-15 | G06F19/00 |
| D,A | WOOLF P J ET AL: "A FUZZY LOGIC APPROACH TO ANALYZING GENE EXPRESSION DATA" PHYSIOLOGICAL GENOMICS, AMERICAN PHYSIOLOGICAL SOCIETY, BETHESDA, MD, US, no. 3, 2000, pages 9-15, XP002909783 ISSN: 1094-8341 * page 9, left-hand column, line 19 - page 10, left-hand column, line 37 * * page 10, right-hand column, line 15 - page 11, left-hand column, line 32 * | 1-15 | TECHNICAL FIELDS SEARCHED (Int.Cl.7) G06F |

-/--

The present search report has been drawn up for all claims

| Place of search | Date of completion of the search | Examiner |
|---|---|---|
| MUNICH | 23 August 2002 | Barba, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

**EUROPEAN SEARCH REPORT**

Application Number

EP 02 01 1654

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | EISEN M B ET AL: "Cluster analysis and display of genome-wide expression patterns" PROCEEDINGS OF THE NATIONAL ACADEMY OF SCIENCES OF USA, NATIONAL ACADEMY OF SCIENCE. WASHINGTON, US, vol. 95, December 1998 (1998-12), pages 14863-14868, XP002140966 ISSN: 0027-8424 * page 14863, left-hand column, line 1 - line 16 * * page 14863, left-hand column, line 47 - page 14864, right-hand column, line 31 * | 1-15 | |
| L | HONGQUAN XU ET AL: "A Smooth Response Surface Algorithm for Constructing Gene Regulatory Network" PREPRINT, 'Online! 2001, pages 1-30, XP002210924 Retrieved from the Internet: <URL:http://preprint.stat.ucla.edu> 'retrieved on 2002-08-23! * the whole document * | 1-15 | TECHNICAL FIELDS SEARCHED (Int.Cl.7) |

The present search report has been drawn up for all claims

| Place of search | Date of completion of the search | Examiner |
|---|---|---|
| MUNICH | 23 August 2002 | Barba, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)